# EUROPEAN PATENT SPECIFICATION

(11) **EP 2 042 127 B1**
(45) Date of publication and mention of the grant of the patent: **16.11.2011**
(21) Application number: 08165095.4
(22) Date of filing: 25.09.2008
(51) Int. Cl.: A61F 2/30, A61F 2/38

(54) **Knee orthopaedic implant**
Orthopädisches Knieimplantat
Implant orthopédique de genou

(30) Priority: 27.09.2007 US 862310
(43) Date of publication of application: 01.04.2009
(73) Proprietor: DePuy Products, Inc., Warsaw, IN 46581 (US)
(72) Inventor: Thomas, Kyle, Ft Wayne, IN 46814 (US); Vendrely, Timothy, Ft Wayne, IN IN 46805 (US)
(74) Representative: Belcher, Simon James

(56) References cited:
- EP-A- 0 947 181
- EP-A- 1 623 686
- WO-A-03/065939
- WO-A-2007/053905
- US-B1- 6 214 052

## Description

This invention relates generally to knee orthopaedic implant devices and more particularly to stem extensions for use with orthopaedic implant devices for knees.

Movement such as flexion and extension of the natural human knee involves movements of the femur and the tibia. Specifically, during flexion and extension, the distal end of the femur and the proximal end of the tibia articulate relative to one another through a series of complex movements. Damage (for example due to trauma) or disease can deteriorate the bones, articular cartilage, and/or ligaments of the knee, which can ultimately affect the ability of the natural knee to function in such a manner. As a result, orthopaedic implant devices have been developed to replace the natural knees of patients.

The longevity of an orthopaedic implant is dependent on a number of factors. One such factor is the proper matching of the implant with the patient's anatomy. Due to the large variation in human anatomy, the surgeon may use orthopaedic implants of various sizes and configurations to ensure proper matching of the implant with the anatomy of the patient.

WO-A-2007/053905 discloses a prosthesis assembly which includes a tray element having an extension member extending from it. A first adaptor fits over the extension member. A second adaptor fits over the free end of the first adaptor and engages a stem. The first adaptor provides for adjustment of the angle of inclination of the stem relative to the tray element. The second adaptor provides for adjustment of the distance between the axis of the stem and the axis of the extension member.

EP-A-1623686 discloses a modular orthopaedic implant system. It includes an adapter which fits between an articulating component of a joint prosthesis and a stem. The adapter has a bore at each of two opposite ends. The axes of the bores can be parallel to one another and offset. The angle between the axes of the bores can be non-zero.

The present invention provides an orthopaedic implant as defined in claim 1.

In some embodiments, the second mounting end may comprise an aperture that receives a tapered end of the stem extension.

In some embodiments, the knee prosthetic component may be embodied as a tibial tray. The tibial tray may include a platform and a stem extending downwardly from a bottom surface of the platform. The stem may have an aperture defined at the distal end. The first mounting end of the adaptor may be tapered such that the aperture of the stem may receive the first mounting end of the adaptor. The first anatomical plane may be the medial/lateral plane, and the second anatomical plane may be the anterior/posterior plane. For example, the second axis of the second mounting end of the adaptor may be offset from and parallel to the first axis of the first mounting end of the adaptor when viewed in the medial/lateral plane. Additionally, the first axis of the first mounting end of the adaptor and the second axis of the second mounting end of the adaptor may define an angle between them greater than zero degrees when viewed in the anterior/posterior plane.

In other embodiments, the knee prosthetic component may be embodied as femoral component. The femoral component may include a pair of condyles and a platform defined between the condyles. The platform may include an aperture defined in an upper surface. The first mounting end of the adaptor may be tapered such that the aperture of the platform may receive the first mounting end of the adaptor. The first anatomical plane may be the anterior/posterior plane, and the second anatomical plane may be the medial/lateral plane. For example, the second axis of the second mounting end of the adaptor may be offset from and parallel to the first axis of the first mounting end of the adaptor when viewed in the anterior/posterior plane. Additionally, the first axis of the first mounting end of the adaptor and the second axis of the second mounting end of the adaptor may define an angle between them which is greater than zero degrees when viewed in the medial/lateral plane.

The adaptor is formed from two pieces. The first piece includes the first mounting end and a first mounting surface. The second piece may includes the second mounting end and a second mounting surface. The second mounting surface is movable relative to the first mounting surface to change the angle defined between the first axis and the second axis when viewed in the second anatomical plane. Additionally, in some embodiments, the first mounting surface may include a first number of teeth and the second mounting surface may include a second number of teeth. The second number of teeth may be interdigitated with the first number of teeth of the first mounting surface.

In some embodiments, the first anatomical plane may be the medial/lateral plane. In other embodiments, the first anatomical plane may be the anterior/posterior plane. For example, the second axis may be offset from and parallel to the first axis by a distance of about 2 mm to about 8 mm when viewed in the medial/lateral plane in some embodiments. In other embodiments, the second axis may be offset from and parallel to the first axis when viewed in the anterior/posterior plane.

In some embodiments, the angle defined between the first axis and the second axis may be greater than or equal to one degree when viewed in the second anatomical plane. In some embodiments, the second anatomical plane may be the anterior/posterior plane. In other embodiments, the second anatomical plane may be the medial/lateral plane. For example, the angle defined between the first axis and the second axis may be about one degree to about five degrees when viewed in the anterior/posterior plane. In other embodiments, the angle defined between the first axis and the second axis may be about one degree to about nine degrees when viewed in the medial/lateral plane. The angle defined between the first axis and the second axis is adjustable when viewed in the second anatomical plane. For example, the elongated shaft may be movable relative to the mounting end to adjust the angle defined between the first axis and the second axis when viewed in the anterior/posterior plane.

Embodiments of the invention are described below by way of example with reference to the accompanying drawings, in which:
FIG. 1 is an exploded view of an orthopaedic implant for use with the tibia of a patient as viewed in the medial/lateral plane;
FIG. 2 is an exploded view of the orthopaedic implant of FIG. 1 as viewed in the anterior/posterior plane;
FIG. 3 is a perspective view of the orthopaedic implant of FIGS. 1 and 2;
FIG. 4 is an exploded view of another orthopaedic implant for use with the femur of a patient as viewed in the medial/lateral plane;
FIG. 5 is an exploded view of the orthopaedic implant of FIG. 4 as viewed in the anterior/posterior plane;
FIG. 6 is a perspective view of the orthopaedic implant of FIGS. 4 and 5;
FIG. 7 is a side elevation view of another stem extension of an orthopaedic implant as viewed in the medial/lateral plane;
FIG. 8 is a side elevation view of the stem extension of FIG. 7 as viewed in the anterior/posterior plane;
FIG. 9 is a perspective view of an adaptor which could be used in accordance with the invention in the orthopaedic implant of FIGS. 1 to 6;
FIG. 10 is an exploded perspective view of the adaptor of FIG. 9;
FIG. 11 is a side elevation view of the adaptor of FIGS. 9 and 10 as viewed in the anterior/posterior plane;
FIG. 12 is an exploded side elevation view of a multiple-piece stem extension as viewed in the medial/lateral plane; and
FIG. 13 is a side elevation view of the stem extension of FIG. 12 as viewed in the anterior/posterior plane.

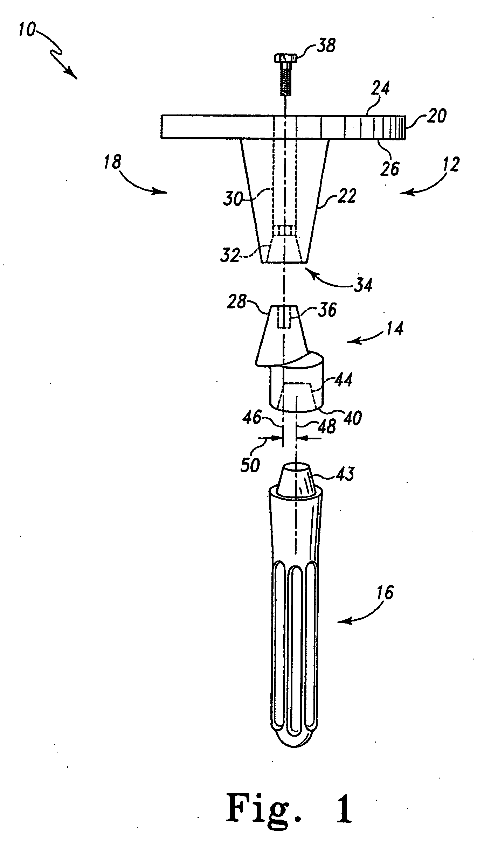

FIGS. 1 to 8 show implant systems which do not include features of the present invention which enable the angle between the first and second axes to be changed. These drawings are retained in this specification to aid understanding of the invention.

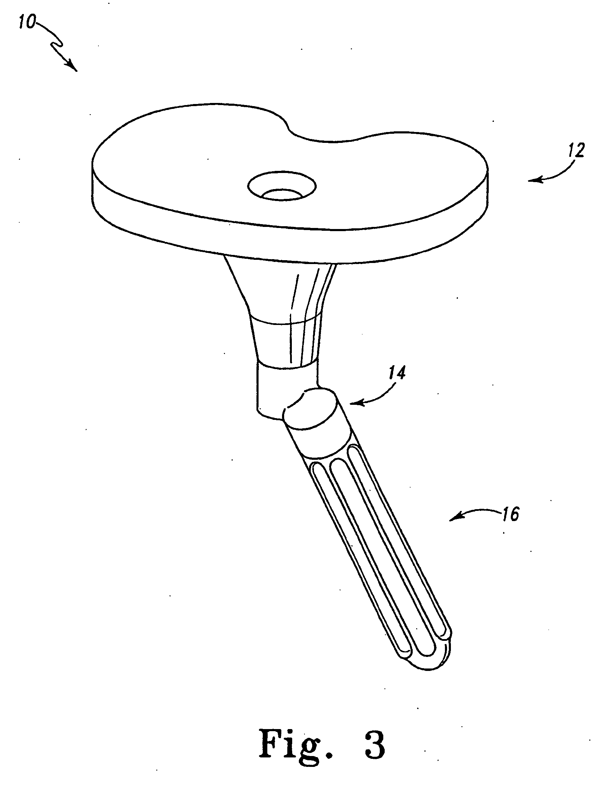

Referring to the drawings, FIGS. 1 to 6 show an orthopaedic implant 10 which includes a knee prosthetic component 12, an adaptor 14, and a stem extension 16. The knee prosthetic component 12, the adaptor 14, and the stem extension 16 are configured to be coupled together as shown in FIG. 3. As described in more detail below, the adaptor 14 is configured to be coupled to both the knee prosthetic component 12 and the stem extension 16. Preferably, the knee prosthetic component 12, the adaptor 14, and the stem extension 16 are each formed from an implantable metallic material such as titanium or cobalt chromium but may be formed from other materials, such as a ceramic, a polymer, a bioengineered, or another like material.

The knee prosthetic component 12 may be embodied as any type of knee prosthetic component configured to receive a stem extension. For example, as shown in FIGS. 1 to 3, the knee prosthetic component 12 may be embodied as a tibial tray 18. The tibial tray includes a platform 20 and a stem 22. The platform 20 includes an upper surface 24 and a lower surface 26. The stem 22 extends downwardly from the lower surface 26. The tibial tray 18 is configured to be coupled to a surgically prepared surface of the proximal end of a patient's tibia (not shown). When the tibial tray 18 is coupled to the patient's tibia, the stem 22 (as well as the adaptor 14 and stem extension 16) is embedded in the patient's tibia to thereby secure the tibial tray 18 to the patient's bone.

The tibial tray 18 is configured to be coupled with a tibial bearing (not shown) such as a polymer tibial bearing. As such, the upper surface 24 of the platform 20 may be configured to receive the tibial bearing. For example, the platform 20 may include any number of rails, tracks, openings, and/or the like configured to receive or otherwise be coupled with a portion of the tibial bearing. Additionally, the tibial tray 18 may be configured to be coupled to any type of tibial bearing, including, for example, a mobile tibial bearing and/or a fixed tibial bearing.

As discussed above, the adaptor 14 is configured to be coupled with the tibial tray 18 and the stem extension 16. As such, the adaptor 14 includes a mounting end 28 configured to be coupled to the tibial tray 18. The mounting end 28 may include any number of apertures, protrusions, grooves, and/or other structures necessary to facilitate the securing of adaptor 14 to the tibial tray 18. For example, in the device shown FIGS. 1 to 3, the stem 22 of the tibial tray 18 includes an internal passage 30 and an aperture 32 defined at a distal end 34. Accordingly, the mounting end 28 of the adaptor 14 is tapered and sized to be received in the aperture 32 of the stem 22. In addition, the mounting end 28 includes a threaded aperture 36. A bolt 38 is threaded into the threaded aperture 36 of the mounting end 28 via the internal passageway 30 of the stem 22 to secure the adaptor 14 to the tibial tray 18.

In other devices, the mounting end 28 of the adaptor 14 may include other or additional structures to facilitate the coupling of the adaptor 14 to the tibial tray 18. For example, the mounting end 28 may include a threaded stud extending therefrom: In such devices, the distal end 34 of the stem 22 may include a corresponding threaded aperture configured to receive the threaded stud of the mounting end 28. Alternatively, the distal end 34 of the stem 22 may be threaded and configured to be received in a threaded aperture defined in the mounting end 28 of the adaptor 14.

The adaptor 14 also includes a mounting end 40 configured to be coupled with the stem extension 16. Similar to the mounting end 28, the mounting end 40 may include any number of apertures, protrusions, grooves, and/or other structures necessary to facilitate the securing of adaptor 14 to the stem extension 16. For example, in the devices shown in FIGS. 1 to 3, the stem extension 16 includes a tapered end 43. Accordingly, the mounting end 40 of the adaptor 14 includes an aperture 44 in which the tapered end 43 of the stem extension 16 is received. Again, in other devices, the mounting end 40 of the adaptor 14 may include other or additional structures to facilitate the coupling of the adaptor 14 to the stem extension 16. For example, the mounting end 40 of the adaptor 14 may include a threaded stud and the end 43 of the stem extension 16 may include a corresponding threaded aperture. In other devices, the end 43 of the stem extension 16 may include a threaded stud and the mounting end 40 of the adaptor 14 may include a corresponding threaded aperture.

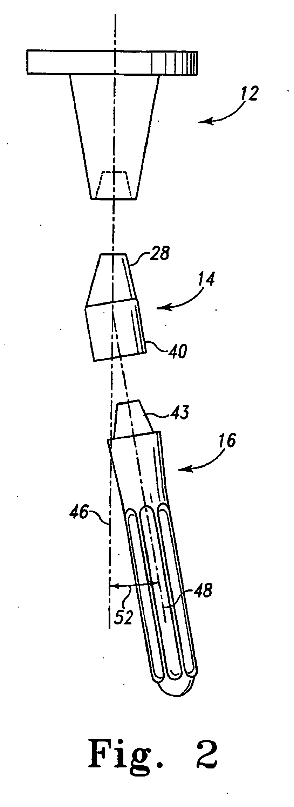

The adaptor 14 is configured such that the position of the stem extension 16 relative to the tibial tray 18 is altered when the adaptor 14 is coupled to them. As shown in FIGS. 1 and 2, the mounting ends 28, 40 define an axis 46 and an axis 48, respectively. The axis 48 defined by the mounting end 40 is offset from and parallel to the axis 46 defined by the mounting end 28 by a distance 50 when the axes 46, 48 are viewed in the medial/lateral plane (see FIG. 1). Additionally, an angle 52 is defined between the axes 46, 48 when the axes 46, 48 are viewed in the anterior/posterior plane (see FIG. 2). It should be appreciated that the terms "medial/lateral plane" and "anterior/posterior plane" have well-understood meanings in the art of orthopaedic implants. As used herein, these terms refer to the respective anatomical planes of a patient when the orthopaedic implant 10 is implanted in the patient.

The adaptor 14 may be configured such that the axis 48 is offset from and angled relative to the axis 46 by any amount suitable for implantation. For example, the axis 48 is offset from the axis 46 by a distance 50 of about two millimeters to about eight millimeters when the axes 46, 48 are viewed in medial/lateral plane (see FIG. 1). Additionally, the axes 46, 48 define an angle 52 between them which is greater than 1°. For example, the angle 52 defined between the axes 46, 48 is in the range of about1° to about 5°. It should be appreciated, however, that other amounts of offset and angulation may be used in other devices based on the particular implementation, bone anatomy of the patient, and the like.

It should be also appreciated that, in use, an orthopaedic surgeon may select an adaptor 14 having a desirable offset and angulation for the particular patient. For example, as shown in FIGS. 1 to 3, because the stem extension 16 is offset in the medial/lateral plane and angled in the anterior/posterior plane when the adaptor 14 is used, the stem extension 16 may be positioned in an improved position for implantation in the particular patient. Additionally, it should be appreciated that the adaptor 14 may be used with an orthopaedic implant configured for the left knee joint or the right knee joint of the patient. Depending on the particular knee joint to be replaced, the adaptor 14 may be secured to the tibial tray 18 such that the offset and angle of the stem extension 16 is in the correct direction and/or anatomical plane for the particular knee joint (e.g., for the left or right knee joint of the patient).

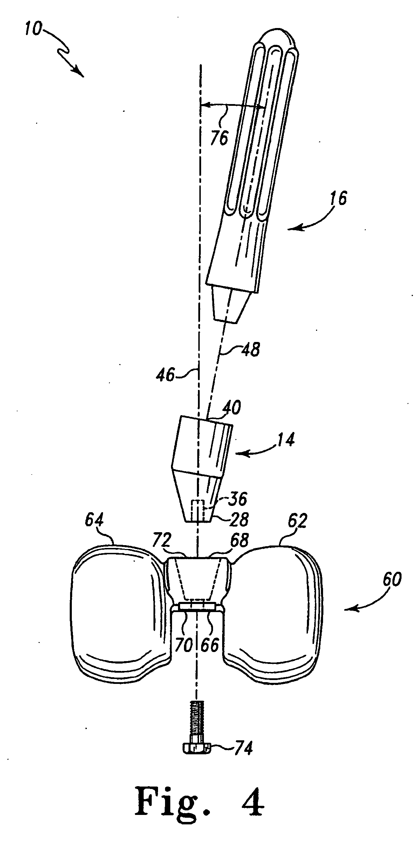

It should be also appreciated that the adaptor 14 may be used with any one of a number of knee prosthetic components and stem extensions to offset the stem extension 16 in a first anatomical plane and angle the stem extension 16 in a second anatomical plane, which is orthogonal to the first anatomical plane. That is, in some devices, the adaptor 14 may be used to offset the stem extension 16 in the medial/lateral plane and angle the stem extension 16 in the anterior/posterior plane (see FIGS. 1 to 3). Alternatively, the adaptor 14 may be configured to offset the stem extension 16 in the anterior/posterior plane and angle the stem extension 16 in the medial/lateral plane. For example, as shown in FIGS. 4 to 6, the adaptor 14 may be used with the knee prosthetic component 12 to offset the stem extension 16 in the anterior/posterior plane and angulate the stem extension 16 in the medial/lateral plane.

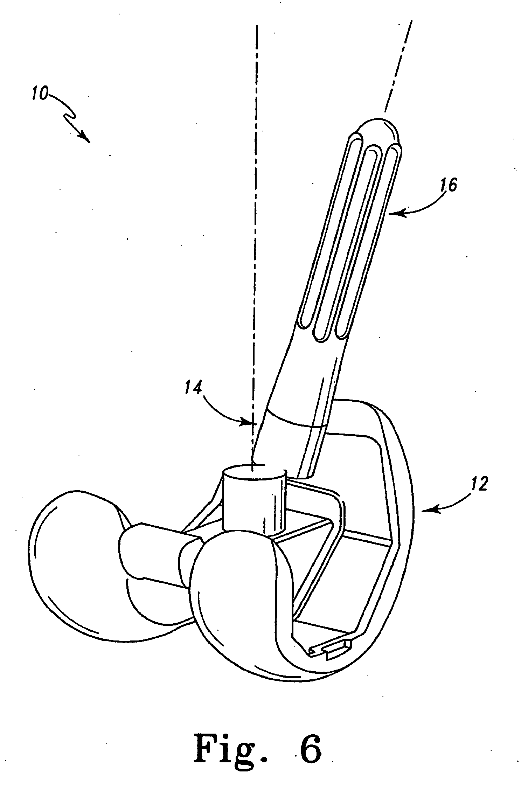

Although the knee prosthetic component 12 is embodied as a tibial tray in the device shown in FIGS. 1 to 3, the knee prosthetic component 12 may be embodied as other types of knee prosthetic components. For example, as shown in FIGS. 4 to 6, the knee prosthetic component 12 may be embodied as a femoral component 60. Similar to the device shown in FIGS. 1 to 3, the femoral component 60, the adaptor 14, and the stem extension 16 are configured to be coupled to together as shown in FIG. 6. The femoral component 60 includes a pair of condyles 62, 64 and a platform 66 defined between the condyles 62, 64. The platform 66 includes an upper surface 68 and a lower surface 70.

The femoral component 60 is configured to be coupled to a surgically prepared surface of the distal end of a patient's femur (not shown). When the femoral component 60 is coupled to the patient's femur, the adaptor 14 and the stem extension 16 are embedded in the patient's femur to thereby secure the femoral component 60 to the patient's bone. In use, the condyles 62, 64 replace the natural condyles of the patient's femur and are configured to articulate on the proximal end of the patient's natural or surgically prepared tibia.

As discussed above, the adaptor 14 is configured to be coupled with the femoral component 60 and the stem extension 16. As such, as described above with reference to FIGS. 1 to 3, the mounting end 28 may include any number of apertures, protrusions, grooves, and/or other structures necessary to facilitate the securing of the adaptor 14 to the femoral component 60. For example, in the device shown in FIGS. 4 to 6, the mounting end 28 is configured to be coupled to the upper surface 68 of the platform 66 of the femoral component 60. To do so, a bolt 74 or other securing device may be inserted through an aperture 72 defined in the platform 66 of the femoral component 60 and threaded into the threaded aperture 36 of the mounting end 28 to secure the adaptor 14 to the femoral component 60. The mounting end 28 may or may not be tapered.

In other devices, the mounting end 28 of the adaptor 14 may include other or additional structures to facilitate the coupling of the adaptor 14 to the femoral component 60. For example, the femoral component 60 may include a stem (not shown) extending upwardly from the upper surface 68 of the platform 66. In such devices, the mounting end 28 may be configured to be coupled to the stem of the femoral component 60. For example, the mounting end 28 may be tapered and sized to be received in an aperture of the stem of the femoral component 60. In other devices, the mounting end 28 may include a threaded stud extending thereform and configured to be received in a corresponding threaded aperture defined in the end of the stem of the femoral component 60. Alternatively, the distal end of the stem of the femoral component 60 may be threaded and configured to be received in a threaded aperture defined in the mounting end 28 of the adaptor 14.

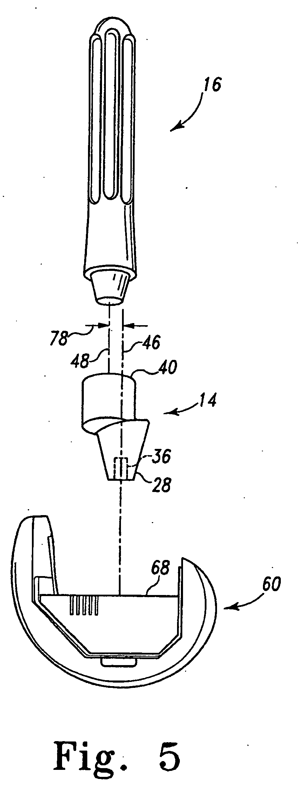

As discussed above with reference to FIGS. 1 to 3, the adaptor 14 also includes the mounting end 40 configured to be coupled with the stem extension 16. The mounting end 40 may include any number of apertures, protrusions, grooves, and/or other structures necessary to facilitate the securing of the adaptor 14 and the stem extension 16. Again, when the femoral component 60, the adaptor 14, and the stem extension 16 are coupled together, the position of the stem extension 16 is altered. In contrast with the device shown in FIGS. 1 to 3, the adaptor 14 is coupled with the femoral component 60 and stem extension 16 such that the axis 48 defined by the mounting end 40 is angled with respect to the axis 46 defined by the mounting end 28 when the axes 46, 48 are viewed in the medial/lateral plane (see FIG. 4). Additionally, the axis 48 defined by the mounting end 40 is offset from and parallel to the axis 46 defined by the mounting end 28 when the axes 46, 48 are viewed in the anterior/posterior plane (see FIG. 5).

The adaptor 14 may be configured such that the axis 48 is offset from and angled relative to the axis 46 by any amount suitable for implantation. For example, the angle 76 defined between the axes 46, 48 is in the range of about 1° to about 9° (see FIG. 4). Additionally, the axis 48 is offset from the axis 46 by the distance 78 of about 2 mm to about 8 mm when the axes 46, 48 are viewed in the anterior/posterior plane (see FIG. 5). Again, it should be appreciated that other amounts of offset and angulation may be used based on the particular implementation, bone anatomy of the patient, and the like.

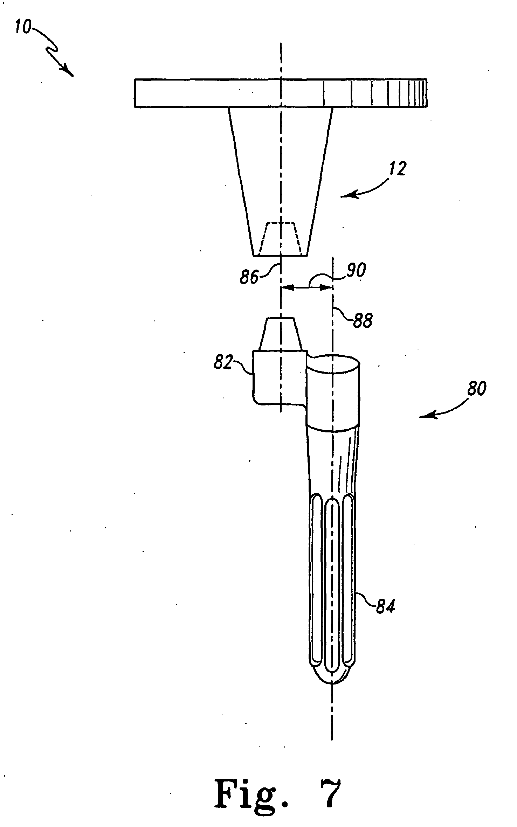

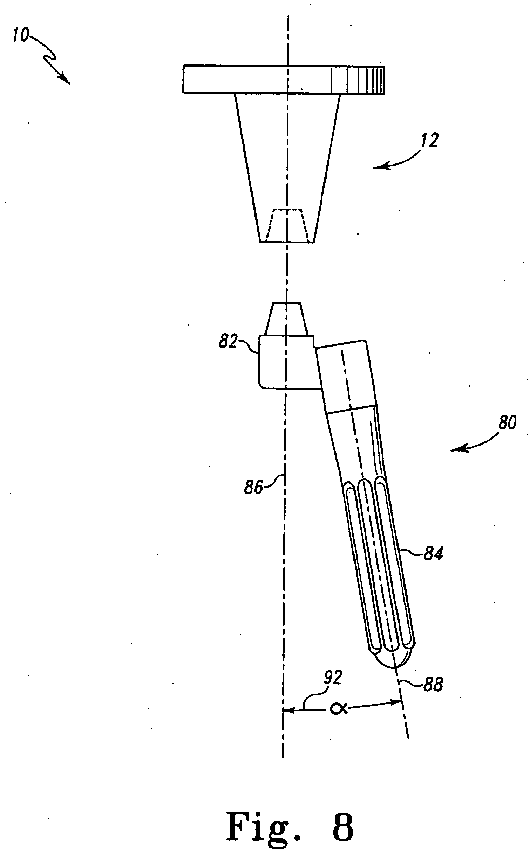

Although the adaptor 14 and the stem extension 16 are shown in FIGS. 1 to 6 as separate pieces, the adaptor 14 and the stem extension 16 may be integral to each other. For example, as shown in FIGS. 7 and 8, the orthopaedic implant 10 may include the knee prosthetic component 12 and a stem extension 80. The stem extension 80 is configured to be coupled with the knee prosthetic component 12. The knee prosthetic component 12 may be embodied as a tibial tray, a femoral component, or any type of knee prosthetic component configured to receive a stem extension. As such, the stem extension 80 includes a mounting end 82 and an elongated shaft 84. The elongated shaft 84 extends from the mounting end 82. Additionally, the mounting end 82 is configured to be coupled to the knee prosthetic component 12. The mounting end 82 may include any number of apertures, protrusions, grooves, and/or other structures necessary to facilitate the securing of stem extension 80 to the knee prosthetic component 12.

The stem extension 80 is configured such that the position of the elongated shaft 84 is altered relative to the knee prosthetic component 12. As shown in FIGS. 7 and 8, the mounting end 82 defines an axis 86. Additionally, the elongated shaft 84 defines an axis 88. Similar to axes 46, 48 discussed above with reference to FIGS. 1 to 3, the axis 88 defined by the elongated shaft 84 is offset from and parallel to the axis 86 defined by the mounting end 82 by a distance 90 when the axes 86, 88 are viewed in the medial/lateral plane (see FIG. 7). Additionally, an angle 92 is defined between the axes 86, 88 when the axes 86, 88 are viewed in the anterior/posterior plane (see FIG. 8).

Similar to the adaptor 14, the stem extension 80 may be configured such that the axis 88 is offset from and angled relative to the axis 86 by any amount suitable for implantation. For example, the axis 88 is offset from the axis 86 by the distance 90 of about 2 mm to about 8 mm when the axes 86, 88 are viewed in the medial/lateral plane (see FIG. 7). Additionally, the axes 86, 88 define an angle 92 between them which is greater than 1° when the axes 86, 88 are viewed in the anterior/posterior plane (see FIG. 8). For example, the angle 92 defined between the axes 86, 88 is in the range of about 1° to about 5°. In other devices, the angle 92 defined between the axes 86, 88 may be in the range of about 1° to about 9°.

It should be appreciated, however, that other amounts of offset and angulation may be used based on the particular implementation, bone anatomy of the patient, and the like. It should also be appreciated that the stem extension 80 is configured to be used with a particular knee joint, either the left knee joint or right knee joint of the patient. A different stem extension 80 may be required for use with the other knee joint such that the offset and angulation of the elongated shaft 84 is proper for the particular knee joint.

It should also be appreciated that the stem extension 80 can be used with any one of a number of knee prosthetic components to offset the elongated shaft 84 in a first anatomical plane and angle the elongated shaft 84 in a second anatomical plane, which is orthogonal to the first anatomical plane. That is, the stem extension 80 may offset the elongated shaft 84 in the medial/lateral plane and angle the elongated shaft 84 in the anterior/posterior plane (see FIGS. 7 and 8). Alternatively, the stem extension 80 may be configured to offset the elongated shaft 84 in the anterior/posterior plane and angle the elongated shaft 84 in the medial/lateral plane.

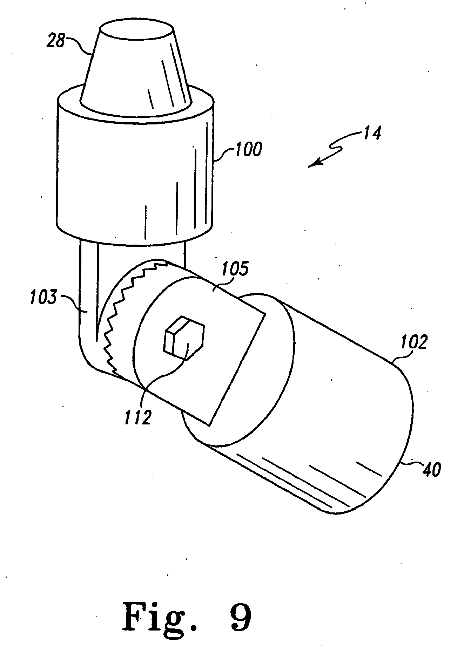

According to the invention, the adaptor 14 is formed from multiple pieces. For example, as shown in FIGS. 9 to 11, the adaptor 14 of FIGS. 1 to 6 may be embodied as a first or knee prosthetic component piece 100 and a second or stem extension piece 102 removably coupled to each other. In addition, the first piece 100 is configured to be coupled to the knee prosthetic component 12. Similarly, the second piece 102 is also configured to be coupled to the stem extension 16.

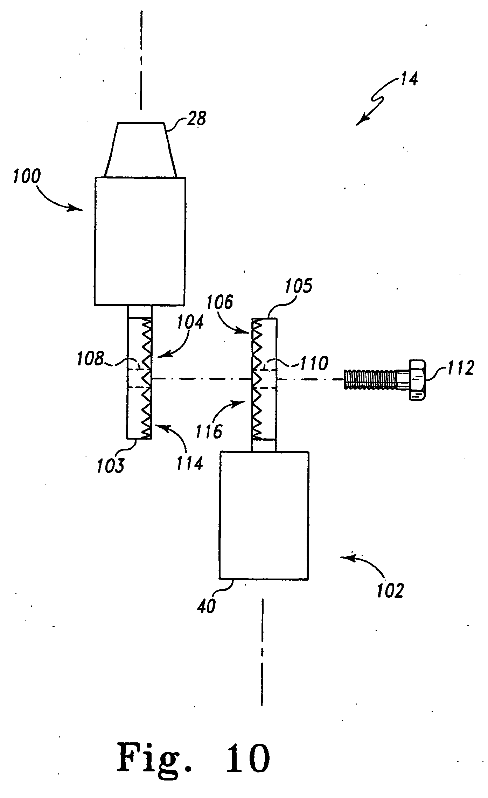

In such embodiments, the first piece 100 of the adaptor 14 includes the mounting end 28 and a mounting plate 103 having a mounting surface 104. The second piece 102 of the adaptor 14 includes the mounting end 40 and a mounting plate 105 having a mounting surface 106. When the pieces 100, 102 of the adaptor 14 are coupled together, the mounting surfaces 104, 106 confront or abut each other. As such, the mounting surfaces 104, 106 may include any number of apertures, protrusions, grooves, and/or other structures necessary to facilitate the securing of the first piece 100 to the second piece 102. For example, referring to FIG. 10, the mounting plate 103 of the first piece 100 includes a threaded aperture 108. The mounting plate 105 of the second piece 102 includes a hole 110. A bolt 112 is threaded into the hole 110 of mounting surface 106 and the threaded aperture 108 of mounting surface 104 to secure piece 102 to piece 100. Additionally, the mounting surface 104 includes a number of teeth 114. Similarly, the mounting surface 106 includes a number of teeth 116 that interdigitate with the number of teeth 114 of mounting surface 104 when the piece 102 is secured to the piece 100.

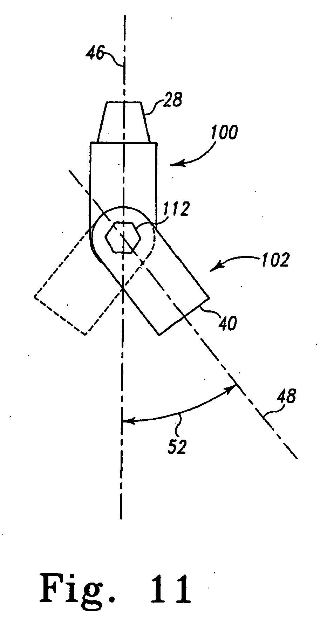

In some embodiments, the second piece 102 may be coupled to the first piece 100 at any one of a number of positions such that the angle 52 (see FIG. 11) defined between the axes 46, 48 when the axes 46, 48 are viewed in the anterior/posterior plane is adjustable. In other embodiments, the second piece 102 may be coupled to the first piece 100 at any one of a number of positions such that the angle defined between the axes 46, 48 when the axes 46, 48 are viewed in the medial/lateral plane is adjustable. Due to the interaction of the number of teeth 114, 116, the position of the second piece 102 of the adaptor 14 relative to the first piece 100 may be adjusted in a stepwise manner. Once the desired position as been reached, the pieces 100, 102 may be secured to each other via the bolt 112.

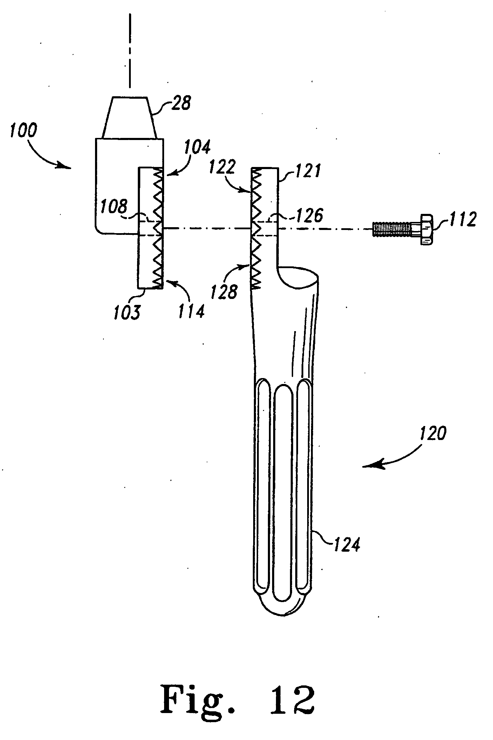

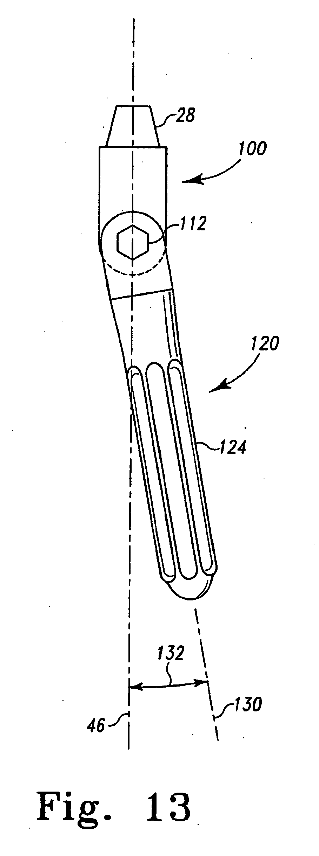

Alternatively, as shown in FIGS. 12 and 13, the second piece 102 of the adaptor 14 may be integral with the stem extension 16. That is, the first piece 100 of the adaptor 14 may be configured to be removably coupled to a stem extension 120. The stem extension 120 includes a mounting plate 121 having a mounting surface 122 and an elongated shaft 124 extending from the mounting plate 121. When the stem extension 120 and the first piece 100 are coupled together, the mounting surfaces 104, 122 confront or abut each other. As such, as described above with reference to FIGS. 9 to 11, the mounting surfaces 104, 122 may include any number of apertures, protrusions, grooves, and/or other structures necessary to facilitate the securing of the stem extension 120 to the first piece 100. For example, referring to FIG. 12, the mounting plate 121 of the stem extension 120 includes a hole 126. A bolt 112 is threaded into the hole 126 of mounting surface 122 and the threaded aperture 108 of the mounting surface 104 to secure the stem extension 120 to the piece 100. Additionally, the mounting surface 122 of the stem extension 120 may include a number of teeth 128 that interdigitate with the number of teeth 114 of mounting surface 104 when the stem extension 120 is secured to the piece 100.

The stem extension 120 may be coupled to the first piece 100 at any one of a number of positions such that an angle 132 (see FIG. 13) defined between the axis 46 defined by the mounting end 28 and an axis 130 defined by the elongated shaft 124 when the axes 46, 48 are viewed in the anterior/posterior plane is adjustable. The stem extension 120 may be coupled to the first piece 100 at any one of a number of positions such that an angle defined between the axis 46 defined by the mounting end 28 and an axis 130 defined by the elongated shaft 124 when the axes 46, 48 are viewed in the medial/lateral plane is adjustable. Due to the interaction of the number of teeth 114, 128, the position of the stem extension 120 relative to the first piece 100 may be adjusted in a stepwise manner. Once the desired position as been reached, the first piece 100 and the stem extension 120 may be secured to each other via the bolt 112.

## Claims

1. An orthopaedic implant (10) comprising:
a knee prosthetic component (12) configured to be coupled to a surgically prepared surface of a bone of a patient, and
an adaptor (14) including a first mounting end (28) configured to be coupled with the knee prosthetic component and a second mounting end (40) configured to be coupled with a stem extension (16), the first mounting end defining a first axis (46) and the second mounting end defining a second axis (48),
in which (i) the second axis is offset from and parallel to the first axis when viewed in a first anatomical plane, and (ii) the first axis and the second axis define an angle between them which is greater than zero degrees when viewed in a second anatomical plane substantially orthogonal to the first anatomical plane,
**characterised in that** the adaptor comprises a first piece (100) including the first mounting end (28) and a first mounting surface (104), and a second piece (102) including the second mounting end (40) and a second mounting surface (106), in which the second piece can be removably coupled to the first piece with the second mounting surface facing the first mounting surface, the second mounting surface being movable relative to the first mounting surface to change the angle defined between the first axis and the second axis when viewed in the second anatomical plane.

2. The orthopaedic implant of claim 1, in which the knee prosthetic component comprises a femoral component (60), the first anatomical plane is the anterior/posterior plane, and the second anatomical plane is the medial/lateral plane.

3. The orthopaedic implant of claim 2, in which the angle defined between the first axis (46) and the second axis (48) when viewed in medial/lateral plane is about one degree to about nine degrees.

4. The orthopaedic implant of claim 1, in which the knee prosthetic component comprises a tibial tray (12), the first anatomical plane is the medial/lateral plane, and the second anatomical plane is the anterior/posterior plane.

5. The orthopaedic implant of claim 1, in which the second mounting end (40) is movable relative to the first mounting end (28).

6. The orthopaedic implant of claim 1, in which the first mounting surface (104) includes a first number of teeth (114) and the second mounting surface (106) includes a second number of teeth interdigitated (116) with the first number of teeth of the first mounting surface.

## Patentansprüche

1. Orthopädisches Implantat (10), das aufweist:
eine Knieprothesenkomponente (12), die ausgelegt ist, dass sie an eine chirurgisch präparierte Fläche eines Knochens eines Patienten zu koppeln ist, und
einen Adapter (14), der ein erstes Anbauende (28), das so ausgelegt ist, dass es mit der Knieprothesenkomponente zu koppeln ist, und ein zweites Anbauende (40), das so ausgelegt ist, dass es mit einer Schaftverlängerung (16) zu koppeln ist, umfasst, wobei das erste Anbauende eine erste Achse (46) definiert und das zweite Anbauende eine zweite Achse (48) definiert,
wobei (i) die zweite Achse von der ersten Achse versetzt und parallel zu ihr ist, wenn in einer ersten anatomischen Ebene betrachtet wird, und (ii) die erste Achse und die zweite Achse zwischen sich einen Winkel definieren, der größer als Null Grad ist, wenn in einer zweiten anatomischen Ebene betrachtet wird, die zu der ersten anatomischen Ebene im Wesentlichen orthogonal ist,
**dadurch gekennzeichnet, dass** der Adapter ein erstes Stück (100), das das erste Anbauende (28) und eine erste Anbaufläche (104) umfasst, und ein zweites Stück (102), das das zweite Anbauende (40) und eine zweite Anbaufläche (106) umfasst, aufweist, wobei das zweite Stück entfernbar mit der zweiten Anbaufläche der ersten Anbaufläche zugewandt an das erste Stück gekoppelt werden kann, wobei die zweite Anbaufläche in Bezug auf die erste Anbaufläche bewegbar ist, um den Winkel zu ändern, der zwischen der ersten Achse und der zweiten Achse definiert ist, wenn in der zweiten anatomischen Ebene betrachtet wird.

2. Orthopädisches Implantat nach Anspruch 1, bei dem die Knieprothesenkomponente eine Oberschenkelkomponente (60) aufweist, die erste anatomische Ebene die anterior/posterior-Ebene ist und die zweite anatomische Ebene die mediale/laterale Ebene ist.

3. Orthopädisches Implantat nach Anspruch 2, bei dem der Winkel, der zwischen der ersten Achse (46) und der zweiten Achse (48) definiert ist, wenn in der medial/lateralen Ebene betrachtet wird, ungefähr ein Grad bis ungefähr neun Grad beträgt.

4. Orthopädisches Implantat nach Anspruch 1, bei dem die Knieprothesenkomponente eine Schienbeinplatte (12) aufweist, die erste anatomische Ebene die medial/laterale Ebene ist und die zweite anatomische Ebene die anterior/posterior-Ebene ist.

5. Orthopädisches Implantat nach Anspruch 1, bei dem das zweite Anbauende (40) in Bezug auf das erste Anbauende (28) bewegbar ist.

6. Orthopädisches Implantat nach Anspruch 1, bei dem die erste Anbaufläche (104) eine erste Anzahl Zähne (114) umfasst, und die zweite Anbaufläche (106) eine zweite Anzahl Zähne (116) umfasst, die mit der ersten Anzahl Zähne der ersten Anbaufläche ineinandergreifend angeordnet sind.

## Revendications

1. Implant orthopédique (10), comprenant :
un composant de prothèse de genou (12) qui est configuré de manière à être couplé à une surface préparée chirurgicalement d'un os d'un patient ; et
un adaptateur (14) qui présente une première extrémité de montage (28), configurée pour être couplée au composant de prothèse de genou, et une deuxième extrémité de montage (40), configurée pour être couplée à une extension de tige (16), la première extrémité de montage définissant un premier axe (46), et la deuxième extrémité de montage définissant un deuxième axe (48) ;
dans lequel (i) le deuxième axe est décalé de et parallèle au premier axe lorsque l'on regarde dans un premier plan anatomique, et (ii) le premier axe et le deuxième axe définissent un angle entre eux qui est supérieur à zéro degré lorsque l'on regarde dans un deuxième plan anatomique qui est sensiblement orthogonal au premier plan anatomique,
**caractérisé en ce que** l'adaptateur comprend une première pièce (100) qui présente la première extrémité de montage (28) et une première surface de montage (104), et une deuxième pièce (102) qui présente la deuxième extrémité de montage (40) et une deuxième surface de montage (106), dans lequel la deuxième pièce peut être couplée de façon amovible à la première pièce avec la deuxième surface de montage qui fait face à la première surface de montage, la deuxième surface de montage étant mobile par rapport à la première surface de montage de manière à modifier l'angle qui est défini entre le premier axe et le deuxième axe lorsque l'on regarde dans le deuxième plan anatomique.

2. Implant orthopédique selon la revendication 1, dans lequel le composant de prothèse de genou comprend un composant fémoral (60), le premier plan anatomique est le plan antérieur/postérieur, et le deuxième plan anatomique est le plan médial/latéral.

3. Implant orthopédique selon la revendication 2, dans lequel l'angle qui est défini entre le premier axe (46) et le deuxième axe (48) lorsque l'on regarde dans le plan médial/latéral est d'environ un degré à environ neuf degrés.

4. Implant orthopédique selon la revendication 1, dans lequel le composant de prothèse de genou comprend un plateau tibial (12), le premier plan anatomique est le plan médial/latéral, et le deuxième plan anatomique est le plan antérieur/postérieur.

5. Implant orthopédique selon la revendication 1, dans lequel la deuxième extrémité de montage (40) est mobile par rapport à la première extrémité de montage (28).

6. Implant orthopédique selon la revendication 1, dans lequel la première surface de montage (104) comporte un premier nombre de dents (114) et la deuxième surface de montage (106) comporte un deuxième nombre de dents (116) qui sont inter-digitées avec le premier nombre de dents de la première surface de montage.
